# EUROPEAN PATENT APPLICATION

(11) **EP 4 850 605 A1**
(43) Date of publication of application: **09.12.2026**
(21) Application number: 26182287.8
(22) Date of filing: 01.06.2026
(51) Int. Cl.: A61B 5/22, A61B 5/00, A61B 5/11, A63B 24/00, A63B 22/06

(54) **QUANTITATION OF PHYSIOLOGICAL STRAIN EXPERIENCED DURING CYCLING ACTIVITY**

(30) Priority: 02.06.2025 US 202563816233 P; 21.05.2026 US 202619684213
(71) Applicant: Wahoo Fitness LLC, Atlanta, GA 30342 (US)
(72) Inventor: CASSIN, Mac, Atlanta, 30342 (US); HENDERSON, Neal, Atlanta, 30342 (US)
(74) Representative: Vossius & Partner Patentanwälte Rechtsanwälte mbB

(57) **Abstract**

Physiological strain experienced by a user during a cycling activity is quantified by a computer and derived as a function of measurements of power exerted by the user during the activity. In a method for quantifying the physiological strain, a processor of the computer calculates a first metric indicative of sub-threshold and threshold level aerobic strain, a second metric indicative of suprathreshold level strain, a third metric indicative of anaerobic strain, and a fourth metric indicative of neuromuscular strain experienced by the user during the activity. A display of the computer displays indicia of the first metric, the second metric, the third metric, and the fourth metric. The processor may calculate an intensity density (ID) score for the activity by summation of the first metric, the second metric, the third metric, and the fourth metric and the display may display the ID score to the user.

## Description

### CROSS-REFERENCE TO RELATED APPLICATION

This application claims the benefit of U.S. Provisional Application No. 63/816,233, filed on June 2, 2025, the entire disclosure of which is incorporated herein by reference.

### FIELD

The present disclosure relates to systems and methods of quantifying physiological strain experienced by an individual during a cycling activity.

### BACKGROUND

This section provides background information related to the present disclosure which is not necessarily prior art.

Various methods of quantifying the physical stress, strain, or load experienced by an athlete during an activity have been developed to assist in formulating training plans that optimize performance while minimizing injury risk. Such methods oftentimes consider the duration of the activity and the intensity of the activity, which may be assessed based on measurements of the athlete's heart rate during the activity. For cycling activities, the intensity of an activity may be assessed based on measurements of the power output of the user during the activity. For example, methods of quantifying the physical load experienced by an athlete during a cycling activity have been developed that rely on a comparison of the athlete's power output during the activity relative to their functional threshold power (FTP), a value commonly recognized as being indicative of the maximal steady state power a cyclist can maintain for a duration of about 60 minutes.

One commonly used method for quantifying the physical load experienced by an athlete during a cycling activity is the Training Stress Score (TSS), which is calculated according to the equation below: $TSS = \frac{Duration \left(s\right) \times {NP}^{2}}{FTP \times 3600}$ where the Duration of the activity is measured in seconds, FTP is the functional threshold power of the athlete, and NP is normalized power, which is an exponentially weighted average of the power output of the athlete during the activity.

### SUMMARY

A computer-implemented method for quantitation of physiological strain experienced by a user during a cycling activity, in accordance with one or more aspects of the disclosure, comprises the following steps: (a) receiving, by a processor of a computer, measurements of power exerted by the user during the activity, (b) calculating, by the processor of the computer, a first metric indicative of sub-threshold and threshold level aerobic strain experienced by the user during the activity, (c) calculating, by the processor of the computer, a second metric indicative of suprathreshold level strain experienced by the user during the activity, (d) calculating, by the processor of the computer, a third metric indicative of anaerobic strain experienced by the user during the activity, (e) calculating, by the processor of the computer, a fourth metric indicative of neuromuscular strain experienced by the user during the activity, and (f) displaying, by a display of the computer, indicia of the first metric, the second metric, the third metric, and the fourth metric, where the first, second, third, and fourth metrics are each derived as a function of the measurements of the power exerted by the user during the activity.

The method may further comprise measuring, by a power meter, the power exerted by the user during the activity.

The method may further comprise storing, in memory of the computer, a functional threshold power (FTP) of the user, a maximal aerobic power (MAP) of the user, an anaerobic capacity (AC) power of the user, and a neuromuscular (NM) power of the user. In such case, the first metric may be further derived as a function of the FTP of the user, the second metric may be further derived as a function of the MAP of the user, the third metric may be further derived as a function of the AC power of the user, and the fourth metric may be further derived as a function of the NM power of the user. The FTP of the user may be the maximum steady-state aerobic power that can be maintained by the user for a duration of about 60 minutes, the MAP of the user may be the maximum power that can be maintained by the user for a duration of about 5 minutes, the AC power of the user may be the maximum power that can be maintained by the user for a duration of about 1 minute when fatigued, and the NM power of the user may be the maximum power that can be maintained by the user for a duration of about 5 seconds.

The method may further comprise: (b1) calculating, by the processor of the computer, a first set of power values by averaging the measurements of the power exerted by the user during the activity in a time window having a first duration at each time point in a set of periodic time points during the activity, (c1) calculating, by the processor of the computer, a second set of power values by averaging the measurements of the power exerted by the user during the activity in a time window having a second duration at each time point in the set of periodic time points during the activity, (d1) calculating, by the processor of the computer, a third set of power values by averaging the measurements of the power exerted by the user during the activity in a time window having a third duration at each time point in the set of periodic time points during the activity, and (e1) calculating, by the processor of the computer, a fourth set of power values by averaging the measurements of the power exerted by the user during the activity in a time window having a fourth duration at each time point in the set of periodic time points during the activity, where the first duration is greater than the second duration, the second duration is greater than the third duration, and the third duration is greater than the fourth duration. In embodiments, the first duration may be in a range of 4 minutes to 6 minutes, the second duration may be in a range of 1 minute to 3 minutes, the third duration may be in a range of 10 seconds to 30 seconds, and the fourth duration may be in a range of one (1) second to less than 10 seconds.

The first metric may be derived as a function of the first set of power values. For example, calculating the first metric may comprise (b2) calculating a sum of first metric data points, where a first metric data point is derived as a function of a ratio of a power value in the first set of power values to the FTP of the user and contributes to the first metric when the power value in the first set of power values is within a predefined tolerance of the FTP of the user.

The third metric may be derived as a function of the third set of power values. For example, calculating the third metric may comprise (d2) calculating a set of power differences as a difference between a power value in the third set of power values and a power value in the second set of power values at each time point in the set of periodic time points during the activity, and (d3) calculating a sum of a third metric power component and an anaerobic spike component. The third metric power component may be a sum of third metric power component data points, where a third metric power component data point is derived as a function of a ratio of a power value in the third set of power values to the AC power of the user and contributes to the third metric power component when the power value in the third set of power values is within a tolerance of the AC power of the user. The anaerobic spike component may be a sum of anaerobic spike component data points, where an anaerobic spike component data point is derived as a function of a ratio of a power difference in the set of power differences to the AC power of the user and contributes to the anaerobic spike component when the power difference in the set of power differences is greater than or equal to a predetermined fraction of the AC power of the user.

The method may further comprise receiving, by the processor of the computer, measurements of a cadence of the user during the activity. In aspects, the method may further comprise measuring, by a cadence sensor, the cadence of the user during the activity.

The method may further comprise receiving an input value for the predefined optimal cadence of the user. For example, the input value for the predefined optimal cadence of the user may be received by a user interface of the computer.

The method may further comprise: (c2) calculating, by the processor of the computer, a first set of cadence values by averaging measurements of the cadence of the user during the activity in a time window having the second duration at each time point in the set of periodic time points during the activity, and (e2) calculating, by the processor of the computer, a second set of cadence values by averaging measurements of the cadence of the user during the activity in a time window having the fourth duration at each time point in the set of periodic time points during the activity.

The second metric may be derived as a function of the second set of power values, the first set of cadence values, and the predefined optimal cadence of the user. For example, calculating the second metric may comprise: (c3) calculating a sum of a second metric power component and a second metric cadence component. The second metric power component may be a sum of second metric power component data points, where a second metric power component data point is derived as a function of a ratio of a power value in the second set of power values to the MAP of the user and contributes to the second metric power component when the power value in the second set of power values is greater than the FTP of the user and is within a tolerance of the MAP of the user. The second metric cadence component may be a sum of second metric cadence component data points, where a second metric cadence component data point is derived as a function of a ratio of a power value in the second set of power values to the MAP of the user and contributes to the second metric cadence component when a cadence value in the first set of cadence values is greater than the predefined optimal cadence of the user.

The method may further comprise receiving, by the processor of the computer, measurements of torque generated by the user the user during the activity. In such case, the method may further comprise (e3) calculating a set of torque values by averaging the torque generated by the user during the activity in a time window having the fourth duration at each time point in the set of periodic time points during the activity.

The method may further comprise calculating, by the processor of the computer, a torque threshold of the user as a function of the predefined optimal cadence of the user and the FTP of the user.

The fourth metric may be derived as a function of the fourth set of power values, the second set of cadence values, the predefined optimal cadence of the user, the set of torque values, and the torque threshold of the user. For example, calculating the fourth metric may comprise: (e4) calculating a sum of a fourth metric power component, a fourth metric cadence component, and a fourth metric torque component. The fourth power component may comprise a sum of fourth metric power component data points, where a fourth metric power component data point is derived as a function of a ratio of a power value in the fourth set of power values to the NM power of the user and contributes to the fourth metric power component when the power value in the fourth set of power values is greater than a predetermined fraction of the NM power of the user. The fourth metric cadence component may comprise a sum of fourth metric cadence component data points, where a fourth metric cadence component data point is derived as a function of a ratio of a power value in the fourth set of power values to the NM power of the user and contributes to the fourth metric cadence component when a cadence value in the second set of cadence values is greater than the optimal cadence of the user. The fourth metric torque component may comprise a sum of fourth metric torque component data points, where each fourth metric torque component data point is derived as a function of a ratio of a power value in the fourth set of power values to the NM power of the user and contributes to the fourth metric torque component when (i) a torque value in the set of torque values at a given time point in the set of periodic time points is greater than the threshold torque of the user and (ii) a cadence value in the second set of cadence values at the given time point is within a tolerance of the predefined optimal cadence of the user or is less than the predefined optimal cadence of the user.

In embodiments, calculating the first metric may further comprise (b3) multiplying the sum of first metric data points by a first multiplier, calculating the second metric may further comprise (c4) multiplying the sum of second metric data points by a second multiplier, calculating the third metric may further comprise (d4) multiplying the sum of third metric data points by a third multiplier, and calculating the fourth metric may further comprise (e5) multiplying the sum of fourth metric data points by a fourth multiplier, where the second multiplier is greater than the first multiplier, the third multiplier is greater than the second multiplier, and the fourth multiplier is greater than the third multiplier.

The method may further comprise calculating, by the processor of the computer, an intensity density (ID) score for the activity by summation of the first metric, the second metric, the third metric, and the fourth metric. The ID score may be indicative of overall accumulated physiological strain experienced by the user during the activity.

In embodiments, the method may further comprise displaying, by the display of the computer, the ID score to the user.

In embodiments, the method may further comprise displaying, by the display of the computer, the portion of the ID score respectively attributed to by the first metric, the second metric, the third metric, and the fourth metric.

In the methods described herein, an indication of overall accumulated physiological strain experienced by a user during a cycling activity can be calculated without measuring or receiving measurements of a heart rate of the user during the activity. For example, the first metric, the second metric, the third metric, and the fourth metric calculated in the presently disclosed methods may not be derived as a function of the heart rate of the user during the activity.

A system for quantitation of physiological strain experienced by a user during a cycling activity, in accordance with one or more aspects of the disclosure, comprises: a bicycle, a power meter, a cadence sensor, and a computer including a display, a processor, and memory. The power meter is configured to measure power generated by a user operating the bicycle and to output a signal indicative of the power measured thereby. The cadence sensor is configured to measure a cadence of the user operating the bicycle and to output a signal indicative of the cadence measured thereby. The memory stores instructions for execution by the processor. The processor is configured by the instructions to perform the following steps: (a) receive measurements of power exerted by the user during the activity, (b) calculate a first metric indicative of sub-threshold and threshold level aerobic strain experienced by the user during the activity, (c) calculate a second metric indicative of suprathreshold level strain experienced by the user during the activity, (d) calculate a third metric indicative of anaerobic strain experienced by the user during the activity, (e) calculate a fourth metric indicative of neuromuscular strain experienced by the user during the activity, and (f) command the display to display indicia of the first metric, the second metric, the third metric, and the fourth metric, where the first, second, third, and fourth metrics are each derived as a function of the measurements of the power exerted by the user during the activity.

### DRAWINGS

The drawings described herein are for illustrative purposes only of selected embodiments and not all possible implementations, and are not intended to limit the scope of the present disclosure.
**FIG. 1** is a schematic depiction of a system for quantitation of physiological strain experienced by a user during a cycling activity.
**FIG. 2** is a flowchart depicting steps in a method for quantitation of physiological strain experienced by a user during a cycling activity.
**FIG. 3** is an example plot of Power vs. Time (minutes) for a user during a 1-Hour Cycling Test that may be used to determine a Power Profile of the user.
**FIG. 4A** is a plot of Power (W) vs. Time (minutes) for a first user during Example Cycling Activity 1.
**FIG. 4B** is a plot of Cadence (rpm) vs. Time (minutes) for the first user during Example Cycling Activity 1.
**FIG. 4C** is a plot of Power (%) and Cadence (%) vs. Time (minutes) for the first user during Example Cycling Activity 1, where the Power (solid line) is expressed as a percentage of the user's FTP and the Cadence (dashed line) is expressed as a percentage of the user's Optimal Cadence (OC).
**FIG. 4D** is a stacked area plot depicting contributions of a 1^{st} Metric (210), a 2^{nd} Metric (220), and a 4^{th} Metric (240) to an Intensity Density Score vs. Time (minutes) for Example Cycling Activity 1. The value of the 3^{rd} Metric was zero during Cycling Activity 1.
**FIG. 4E** is an enlarged view of a portion of the plot of **FIG. 4C** and a portion of the stacked area plot of **FIG. 4D** covering one of the low-cadence efforts during Cycling Activity 1.
**FIG. 4F** is an enlarged view of a portion of the plot of **FIG. 4C** and a portion of the stacked area plot of **FIG. 4D** covering one of the high-cadence efforts during Cycling Activity 1.
**FIG. 5A** is a plot of Power (W) vs. Time (minutes) for a first user during Example Cycling Activity 2.
**FIG. 5B** is a plot of Cadence (rpm) vs. Time (minutes) for the first user during Example Cycling Activity 2.
**FIG. 5C** is a plot of Power (%) and Cadence (%) vs. Time (minutes) for the first user during Example Cycling Activity 2, where the Power (solid line) is expressed as a percentage of the user's FTP and the Cadence (dashed line) is expressed as a percentage of the user's OC.
**FIG. 5D** is a stacked area plot depicting contributions of a 1^{st} Metric (210), a 2^{nd} Metric (220), and a 3^{rd} Metric (230) to an Intensity Density Score vs. Time (minutes) for Example Cycling Activity 2. The value of the 4^{th} Metric was zero during Cycling Activity 2.
**FIG. 5E** is an enlarged view of a portion of the plot of **FIG. 5C** and a portion of the stacked area plot of **FIG. 5D** covering one of the intervals during Cycling Activity 2.
**FIG. 6A** is a stacked area plot depicting contributions of a 1^{st} Metric (210), a 2^{nd} Metric (220), and a 3^{rd} Metric (230) to an Intensity Density Score vs. Time (minutes) for Example Cycling Activity 3 and plot of Power (%) and Cadence (%) vs. Time (minutes) for a second user during Example Cycling Activity 3, where the Power (solid line) is expressed as a percentage of the user's FTP and the Cadence (dashed line) is expressed as a percentage of the second user's OC.
**FIG. 6B** is an enlarged view of a portion of the plot of **FIG. 6A** covering one of the intervals during Cycling Activity 3.

Corresponding reference numerals indicate corresponding parts throughout the several views of the drawings.

### DETAILED DESCRIPTION

Example embodiments will now be described more fully with reference to the accompanying drawings.

The presently disclosed methods and systems can be used to calculate a multicomponent Intensity Density (ID) Score that can be used to make a quantitative assessment of the load or physiological strain experienced by an athlete during a cycling activity. The presently disclosed ID Score is derived as a function of certain predetermined performance metrics of the user and measurements of the power output, cadence, and torque of the user during the cycling activity. Specifically, the presently disclosed ID Score is derived as a function of the athlete's functional threshold power (FTP), maximal aerobic power (MAP), anaerobic capacity (AC) power, neuromuscular (NM) power, optimal cadence (OC), and threshold torque (TT), and thus is more individualized and considers more aspects of the athlete's physical capabilities than existing methods of quantifying the physical load of an activity. In addition, because the presently disclosed ID Score is derived as a function of the athlete's cadence and torque during the activity (in addition to the athlete's power output), it is more comprehensive than existing methods of quantifying the physical load of an activity.

The presently disclosed ID Score is a sum of four individually derived metrics that are each specifically formulated to quantify a different type of physiological strain experienced by an athlete during a cycling activity, and thus is more granular than existing methods of quantifying the physical load of an activity. A first metric is formulated to quantify sub-threshold and threshold level aerobic strain experienced by the athlete, which is generally experienced when the average power output of the athlete in a 5-minute time window during the cycling activity is at or below the athlete's FTP. A second metric is formulated to quantify suprathreshold level strain, which is generally experienced by the athlete, when the average power output of the athlete in a 2-minute time window during the cycling activity is at or near the athlete's MAP and/or when the average power output of the athlete in the 2-minute time window is above the athlete's FTP and the athlete is cycling at a higher than optimal cadence. A third metric is formulated to quantify anaerobic strain experienced by the athlete, which is generally experienced when the average power output of the athlete in a 15-second time window during the cycling activity is near or above the athlete's AC Power and/or when a spike in the average power output of the athlete occurred within a 15-second time window during the activity. A fourth metric is formulated to quantify neuromuscular strain experienced by the athlete, which is generally experienced when the average power output of the athlete in a 5-second time window during the cycling activity is near or above the athlete's NM Power, when the average cadence of the athlete in a 5-second time window during the cycling activity is above the athlete's optimal cadence, and/or when the average torque of the athlete in a 5-second time window during the cycling activity is above the athlete's threshold torque and the cadence of the use in the same time window is close to or less than the athlete's optimal cadence.

In order to calculate values for the first, second, third, and fourth metrics for a single cycling activity, moving averages of the power, cadence, and/or torque are calculated for time windows having first, second, third, and fourth durations at periodic intervals during the cycling activity, with the first duration being longer than the second duration, the second duration being longer than the third duration, and the third duration being longer than the fourth duration. The first metric is derived as a function of the moving averages of power calculated in a time window having the longest (i.e., the first duration), with the first duration being selected to provide power values that can accurately serve as a proxy for assessing sub-threshold and threshold level aerobic strain. The second metric is derived as a function of the moving averages of power and cadence calculated in a time window having the second longest (i.e., the second duration), with the second duration being selected to selected to provide power values and cadence values that can accurately serve as a proxy for assessing suprathreshold level strain. The third metric is derived as a function of the moving averages of power calculated in a time window having the third duration, with the third duration being selected to selected to provide power values that can accurately serve as a proxy for assessing anaerobic strain. The fourth metric is derived as a function of the moving averages of power, cadence, and torque calculated in a time window having the shortest (i.e., the fourth duration), with the fourth duration being selected to selected to provide power, cadence, and torque values that can accurately serve as a proxy for assessing neuromuscular strain.

Because the presently disclosed ID Score is a sum of the first, second, third, and fourth metrics, that are respectively derived as a function of moving averages of the power, cadence, and/or torque in time windows having first, second, third, and fourth durations, the presently disclosed ID Score can effectively quantify different types of physiological strain that may be occurring at the same time during an activity. As such, the presently disclosed ID Score is more accurate and comprehensive than methods of quantifying physical load that rely on assessment of the time an athlete spent cycling in a predefined "power zone." In addition, in calculating the ID Score, the value of the first, second, third, and fourth metrics are scaled or weighted relative to one another to account for the fact that different types of physiological strain impart different levels of strain on an athlete, which allows the ID Score to be used to effectively quantify an athlete's overall fatigue, adaptation, and training status.

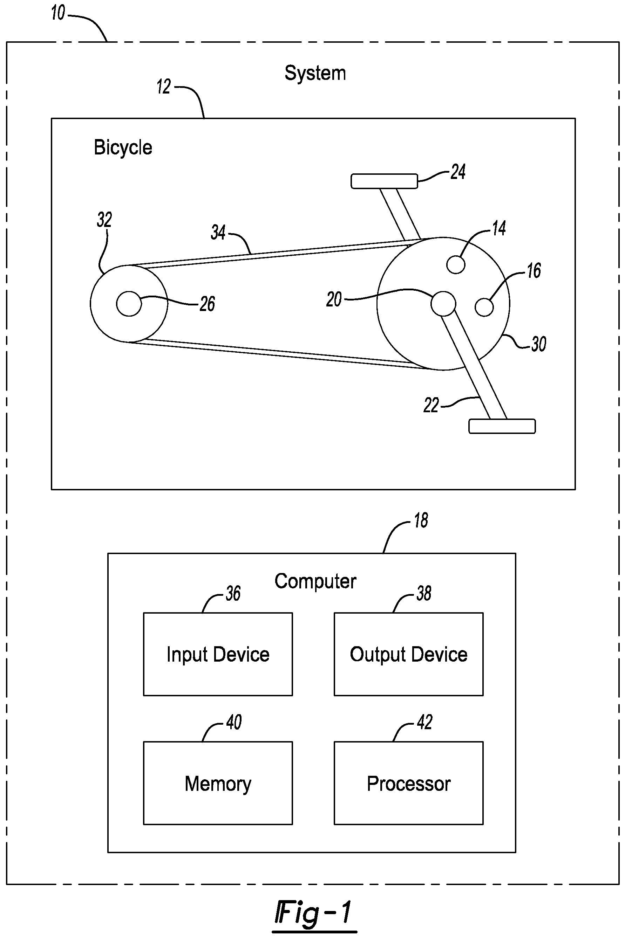

**FIG. 1** depicts a system 10 for quantitation of cumulative physiological strain experienced by a user during an activity. The system 10 includes a bicycle 12, a power meter 14, a cadence sensor 16, and a computer 18.

The bicycle 12 may be any type of crank-powered cycle, including traditional bicycles, hand cycles, and stationary cycles. The bicycle 12 includes a drive axle 20, first and second cranks 22 mounted 180 degrees from each other on opposite ends of the drive axle 20, first and second pedals 24 respectively coupled to the first and second cranks 22, a driven component 26, and a drivetrain 28 coupled to the first and second cranks 22 and to the driven component 26. The drivetrain 28 is configured to transfer rotational forces, i.e., torque, applied to the first and second pedals 24 by a user to the driven component 26. In aspects, the drivetrain 28 may comprise a drive gear 30 coupled to and driven by rotation of the first and second cranks 22 and a driven gear 32 coupled to the driven component 26, for example, via a belt, chain, or other mechanism 34. In aspects where the bicycle 12 is a traditional bicycle having two wheels, the driven component 26 may comprise a rear wheel (now shown) of the bicycle 12 and torque may be transferred by the drivetrain 28 from the first and second cranks 22 to the rear wheel of the bicycle 12 to propel forward movement of the bicycle 12. In aspects where the bicycle 12 is a stationary bicycle or other type of stationary cycling device, the torque may be transferred by the drivetrain 28 from the first and second cranks 22 to a flywheel (not shown).

The power meter 14 may be coupled to the bicycle 12, as shown in **FIG. 1**, and is configured to output a power signal indicative of the power generated by the user during operation of the bicycle 12. In aspects, the power meter 14 may calculate the power generated by the user, for example, based on measurements or calculations of the torque applied to the cranks 22 by the user and an angular velocity of the cranks 22. The torque applied to the cranks 22 may be determined, for example, using one or more strain gages, which may measure the amount of deformation of a spring element.

The cadence sensor 16 may be coupled to the bicycle 12, as shown in **FIG. 1**, and is configured to output a cadence signal indicative of the cadence of the user. In aspects, the cadence sensor 16 may measure or calculate a cadence of the user based on a measurement of the number of revolutions of the cranks 22 per minute.

The power meter 14 and the cadence sensor 16 may be configured to respectively output the power signal and the cadence signal to the computer 18, or to another intermediate device, which may directly or indirectly communicate the power signal and the cadence signal to the computer 18. It is to be understood that, although the power signal and the cadence signal may or may not include data based on direct measurements of the power and/or the cadence of the user, the data included in the power signal and the cadence signal may nonetheless be respectively referred to herein as measurements of the power and the cadence of the user.

The computer 18 is communicatively coupled to the power meter 14 and the cadence sensor 16 associated with the bicycle 12 and is configured to provide to a user of the bicycle 12 a quantitative indication of physiological strain experienced by the user during a given session or activity on the bicycle 12. The computer 18 includes an input device 36, an output device 38, memory 40, and a processor 42.

The input device 36 is configured to receive input signals from one or more devices and/or from the user and to communicate the input signals to the memory 40 and/or the processor 42. For example, the input device 36 is configured to receive a power signal (e.g., from the power meter 14) indicative of the power generated by the user during an activity on the bicycle 12 and a cadence signal (e.g., from the cadence sensor 16) indicative of the cadence of the user during an activity on the bicycle 12. In addition, the input device 36 is configured to receive input signals indicative of various predetermined performance metrics and/or attributes of the user. The input device 36 may be embodied in one or more components of the computer 18. For example, the input device 36 may comprise a wireless receiver, touch screen, buttons, microphone, user interface, wireless input device, and/or data input.

The output device 38 is configured to generate output signals to communicate with the user and/or to another electronic device in response to commands from the processor 42. The output device 38 may be embodied in one or more components of the computer 18. For example, the output device 38 may comprise a display, a touch screen, speakers, data connection, and/or wireless transmitter.

The memory 40 is configured to store user data and computer-executable instructions for execution by the processor 42. The user data stored in the memory 40 may include the predetermined performance metrics and/or attributes of the user received by the input device 36, as well as measurements of the power and/or cadence of the user during an activity performed on the bicycle 12. The memory 40 may be a tangible computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions and/or data, and each coupled to a computer system bus.

The processor 42 is coupled with the input device 36, the output device 38, and the memory 40 and is configured to receive input signals from the input device 36, execute computer-executable instructions stored in the memory 40, and control the output device 38 to communicate certain information to the user, for example, to provide the user with a quantitative indication of physiological strain experienced by the user during a given activity on the bicycle 12. In aspects, the processor 42 may be a single processor or may be architectures employing multiple processor designs for increased computing capability.

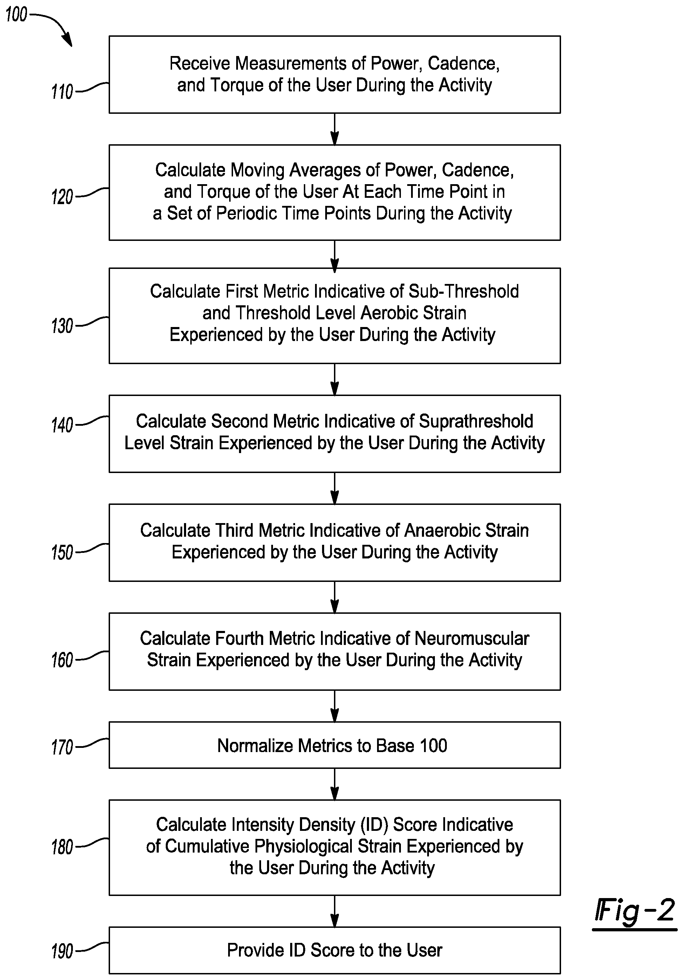

**FIG. 2** depicts a method 100 that can be performed by the computer 18 to provide a user of the bicycle 12 with a quantitative indication of cumulative physiological strain experienced by the user during an activity on the bicycle 12. For example, one of more of the steps of the method 100 may be performed by the processor 42 of the computer 18 and the instructions for performing such steps may be stored in the memory 40 of the computer 18. The method 100 may include one or more of the following steps.

In a first step 110, measurements of the power exerted by the user, the cadence of the user, and/or the torque applied to the cranks 22 of the bicycle 12 during the activity are received by the processor 42 of the computer 18, for example, via the input device 36.

In aspects, the first step 110 may further include receiving or calculating a set of predetermined performance metrics and/or attributes of the user, for example, via the input device 36, and storing the predetermined performance metrics and/or attributes of the user in the memory 40 of the computer 18. The predetermined performance metrics of the user may include functional threshold power (FTP), maximal aerobic power (MAP), anaerobic capacity (AC) power, neuromuscular (NM) power, an optimal cadence (OC), and threshold torque (TT). The FTP of the user is indicative of the maximum steady-state aerobic power that can be maintained by the user for a duration of about 60 minutes and may be calculated based on the maximum power generated by the user during a 20-minute period. The MAP of the user is roughly equivalent to the maximum power that can be maintained by the user when the user is at their VO₂ max (i.e., the user's maximum rate of oxygen consumption attainable during physical exertion) and may be calculated based on the maximum power that can be maintained by the user for a duration of about 5 minutes. The MAP of the user may be affected based the cadence of the user because higher-than-optimal cadences reduce oxygen efficiency and thus may reduce the power at which the user reaches their VO₂ max. The AC power of the user is indicative of the user's ability to generate high power in a short duration, as well as their ability to recover from hard efforts, and may be calculated based on the maximum power the user can maintain for a duration of about 1 minute when fatigued. The NM power of the user is indicative of the user's instantaneous peak power or sprint power, as well as their muscular coordination, and may be calculated based on the maximum power the user can maintain for a duration of about 5 seconds. The FTP, the MAP, the AC power, and the NM power of the user may be collectively referred to herein as the Power Profile of the user and may be obtained via analysis of measurements of the power exerted by the user during one or more previous activities. One method of obtaining the Power Profile of a user is described in the Example entitled Power Profile Assessment set forth herein below.

The optimal cadence of the user is indicative of the cadence associated with a peak efficiency of the user during sustained long duration operation of the bicycle 12. The optimal cadence of the user may be input to the input device 36 by the user or may be calculated, for example, based on the average cadence of the user during one or more portions of the 1-Hour Cycling Test used to obtain their Power Profile, or on the average cadence of the user during the portions of the previous activities used to calculate the FTP of the user. The threshold torque of the user is the value of the torque generated by the user when the user is riding at their optimal cadence and FTP and may be calculated based on the average cadence and the average power of the user during one or more portions of the 1-Hour Cycling Test used to obtain their Power Profile, or on the average cadence of the user during the portions of the previous activities used to calculate the FTP of the user.

In a second step 120, moving averages of the power, cadence, and torque of the user during the activity are calculated by the processor 42 for each time point in a set of periodic time points during the activity. To accomplish this, a set of periodically spaced-apart time points is identified during the activity. The duration between each time point in the set of periodic time points is not particularly limited and may be, for example, 1 second, 3 seconds, or 5 seconds, etc. In aspects, the duration between each time point in the set of periodic time points is 1 second.

The moving averages of the power, cadence, and torque of the user are calculated for time windows having first, second, third, and/or fourth durations at each time point in the set of periodic time points during the activity. The first, second, third, and fourth durations of the time windows used to calculate the moving averages are different from one another. Specifically, the first duration is greater than the second duration, the second duration is greater than the third duration, and the third duration is greater than the fourth duration.

The purpose of calculating averages of the power of the user in time windows having the first duration is to account for steady, moderate-to-low intensity endurance efforts of the user during the activity. In addition, variations in the average power of the user calculated in moving windows having the first duration during the activity are believed to closely align with variations in the heart rate of the user, making averages of the power of the user in time windows having the first duration particularly useful for assessing the aerobic endurance of the user. The first duration may be greater than 3 minutes, or optionally greater than or equal to 4 minutes, and less than or equal to 10 minutes, optionally less than or equal to 8 minutes, or optionally less than or equal to 6 minutes. In aspects, the first duration is about 5 minutes.

The purpose of calculating averages of the power of the user in time windows having the second duration is to account for intense aerobic efforts of the user during the activity. The purpose of calculating averages of the cadence of the user in time windows having the second duration is to account for quick physiological responses by the user, which may require rapid adjustments in oxygen consumption. The second duration may be greater than 30 seconds, optionally greater than or equal to 1 minute, or optionally greater than or equal to 1.5 minutes, and less than or equal to 3 minutes, or optionally less than or equal to 2.5 minutes. In aspects, the second duration is about 2 minutes.

The purpose of calculating averages of the power of the user in time windows having the third duration is to account for short, high-intensity efforts of the user, which impart immediate energy demands on the user and require use of the user's anaerobic energy systems. The third duration may be greater than 10 seconds and less than 30 seconds, or optionally less than or equal to 20 seconds. In aspects, the third duration is about 15 seconds.

The purpose of calculating averages of the power, cadence, and torque of the user in time windows having the fourth duration is to account for brief and intense efforts of the user that require use of the user's neuromuscular systems. In addition, the purpose of calculating averages of the torque of the user in time windows having the fourth duration is to account for varying muscular and oxygen demands at different cadences, even at the same power output. The fourth duration may be greater than or equal to 1 second, or optionally greater than or equal to 3 seconds, and less than or equal to 10 seconds, optionally less than or equal to 8 seconds, or optionally less than or equal to 6 seconds. In aspects, the fourth duration is about 5 seconds. Although the fourth duration is only a matter of seconds, it should be long enough to minimize impacts from data anomalies or brief spikes in the power, cadence, or torque of the user, thereby ensuring an accurate assessment of the user's neuromuscular strain.

In aspects, the moving averages of the power, cadence, and torque of the user are exponentially weighted moving averages (EWMA). In such case, the moving averages may be calculated according to equations (1), (2), (3), and (4): ${α}_{τ} = 1 - {e}^{- Δ t / τ}$ (1) ${Pwr}_{τ} \left(t\right) = {α}_{τ} Pwr \left(t\right) + \left(1-{α}_{τ}\right) {Pwr}_{τ} \left(t-1\right)$ (2) ${Cad}_{τ} \left(t\right) = {α}_{τ} Cad \left(t\right) + \left(1-{α}_{τ}\right) {Cad}_{τ} \left(t-1\right)$ (3) ${Trq}_{τ} \left(t\right) = {α}_{τ} Trq \left(t\right) + \left(1-{α}_{τ}\right) {Trq}_{τ} \left(t-1\right)$ (4) where *τ* is the duration of the time window, tis the time point in the set of periodic time points during the activity, Δ*t* is the duration between each time point in the set of periodic time points, Pwr(t) is the power exerted by the user at time point *t* during the activity, Pwr_{τ}(t) is the EWMA of the power in a time window having a duration *τ* at time point *t* during the activity, Cad(t) is the cadence of the user at time point *t* during the activity, Cad_{τ}(t) is the EWMA of the cadence in a time window having a duration *τ* at time point *t* during the activity, Trq(t) is the torque exerted by the user at time point *t* during the activity, and Trq_{τ}(t) is the EWMA of the torque in a time window having a duration *τ* at time point *t* during the activity.

With respect to the moving averages (e.g., EWMAs) of the power generated by the user during the activity, a first set of power values is calculated by averaging measurements of the power exerted by the user during the activity in a time window having the first duration, a second set of power values is calculated by averaging measurements of the power exerted by the user during the activity in a time window having the second duration, a third set of power values is calculated by averaging measurements of the power exerted by the user during the activity in a time window having the third duration, and a fourth set of power values is calculated by averaging measurements of the power exerted by the user during the activity in a time window having the fourth duration at every time point in the set of periodic time points during the activity.

With respect to the moving averages (e.g., EWMAs) of the cadence of the user during the activity, a first set of cadence values is calculated by averaging measurements of the cadence of the user during the activity in a time window having the second duration and a second set of power values is calculated by averaging measurements of the power exerted by the user during the activity in a time window having the fourth duration at every time point in the set of periodic time points during the activity.

With respect to the moving averages (e.g., EWMAs) of the torque generated by the user during the activity, a set of torque values is calculated by averaging measurements of the torque generated by the user during the activity in a time window having the fourth duration at every time point in the set of periodic time points during the activity.

In a third step 130, a first metric (1^{st} Metric) indicative of sub-threshold and threshold level aerobic strain experienced by the user during the activity is calculated by the processor 42. The 1^{st} Metric quantifies the total physiological strain or load associated with sustained endurance-level power outputs of the user that occur below or very close to the user's FTP during the activity and is specifically designed to reflect cumulative physiological stress from low-to-moderate-intensity endurance efforts during the activity. The 1^{st} Metric is a sum of 1^{st} Metric data points multiplied by a first multiplier, with each 1^{st} Metric data point corresponding to a single time point in the set of periodic time points during the activity. Each 1^{st} Metric data point is derived as a function of a ratio of a power value in the first set of power values to the FTP of the user raised to the power of a first exponent. A 1^{st} Metric data point is only included in the sum of 1^{st} Metric data points, and thus only contributes to the 1^{st} Metric, when the power value in the first set of power values is within a predefined tolerance of the FTP of the user, which ensures most aerobic efforts of the user during the activity are accounted for in the 1^{st} Metric.

In aspects, the 1^{st} Metric is calculated according to equations (5), (6), and (7): $Sum _ 1 {M}_{dpts} = {\sum }_{t} {\left(\frac{{Pwr}_{1} \left(t\right)}{FTP}\right)}^{Exp _ 1} , \left[0\leq {Pwr}_{1}\left(t\right)\leq Pwr_C1\right]$ (5) $Pwr _ C 1 = FTP \times {k}_{Pwr _ C 1}$ (6) $1 st Metric = Sum _ 1 {M}_{dpts} \times Mult _ 1$ (7) where Sum_1M_{dpts} is a sum of 1^{st} Metric data points, Pwr₁(t) is an average (e.g., an EWMA) of the power generated by the user in a time window having the first duration at time point *t* during the activity, Exp_1 is a first exponent, FTP is the FTP of the user, Pwr_C1 is a first power ceiling, k_{Pwr_C1} is a first power ceiling constant, and Mult_1 is a first multiplier. As shown in equation (5), the value of a 1^{st} Metric data point 1M_{dpt} calculated at time *t* is only included in the sum of 1^{st} Metric data points Sum_1M_{dpts} if the power value Pwr₁(t) at time *t* is less than or equal to the FTP of the user multiplied by the first power ceiling constant k_{Pwr_C1}. The first power ceiling constant k_{Pwr_C1} is greater than or equal to 1 and less than or equal to 1.1. In aspects, the first power ceiling constant k_{Pwr_C1} is 1.05. The first exponent Exp_1 and the first multiplier Mult_1 are included in the calculation of the 1^{st} Metric to normalize the value of the 1^{st} Metric in the calculation of the overall physiological strain experienced by the user during the activity by adjusting for the fact that higher power outputs have a greater impact per time unit on overall physiological strain than lower power outputs. The first exponent Exp_1 is a constant and is included in the calculation of the 1^{st} Metric to intensify the weighting of higher power values in the first set of power values and is greater than 1. In aspects, the first exponent Exp_1 is 2. The first multiplier Mult_1 is configured to scale the contributions of the sub-threshold and threshold level aerobic efforts of the user relative to the contributions of the suprathreshold level efforts, anaerobic efforts, and/or neuromuscular efforts of the user during the activity. In aspects, the first multiplier Mult_1 is 4.

In a fourth step 140, a second metric (2^{nd} Metric) indicative of suprathreshold level strain experienced by the user during the activity is calculated by the processor 42. The 2^{nd} Metric quantifies the total physiological strain or load associated with sustained aerobic efforts at or near the user's MAP and considers both the power and cadence of the user during the activity. The 2^{nd} Metric is a sum of 2^{nd} Metric data points multiplied by a second multiplier, with each 2^{nd} Metric data point corresponding to a single time point in the set of periodic time points during the activity. Each 2^{nd} Metric data point only contributes to the 2^{nd} Metric when the average power exerted by the user in a time window having the second duration is greater than the FTP of the user and is within a tolerance of the MAP of the user and/or the average cadence of the user in a time window having the second duration is greater than the optimal cadence of the user.

Each 2^{nd} Metric data point is a sum of a 2^{nd} Metric power component and a 2^{nd} Metric cadence component. The 2^{nd} Metric power component is a sum of 2^{nd} Metric power component data points and the 2^{nd} Metric cadence component is a sum of 2^{nd} Metric cadence component data points, with each power component data point and cadence component data point corresponding to a single time point in the set of periodic time points during the activity. Each 2^{nd} Metric power component data point is derived as a function of a ratio of a power value in the second set of power values to the MAP of the user and only contributes to the 2^{nd} Metric power component when the power value in the second set of power values is greater than the FTP of the user and is within a tolerance of the MAP of the user. Each 2^{nd} Metric cadence component data point is derived as a function of a ratio of a power value in the second set of power values to the MAP of the user multiplied by an efficiency constant and only contributes to the 2^{nd} Metric cadence component when a cadence value in the first set of cadence values is greater than the optimal cadence of the user.

In aspects, the 2^{nd} Metric is calculated according to equations (8), (9), (10), (11), (12), and (13): $Pwr _ 2 M = {\sum }_{t} \left(\frac{{Pwr}_{2} \left(t\right)}{MAP}\right) , \left[Pwr_F2\leq {Pwr}_{2}\left(t\right)\leq Pwr_C2\right]$ (8) $Pwr _ F 2 = max \left\{MAP\times {k}_{Pwr _ B 1},FTP\times {k}_{Pwr _ B 2}\right\}$ (9) $Pwr _ C 2 = MAP \times {k}_{Pwr _ C 2}$ (10) $Cad _ 2 M = {\sum }_{t} \left(\frac{{Pwr}_{2} \left(t\right) \times {k}_{Eff}}{MAP}\right) , \left[{Cad}_{2}\left(t\right)\geq Cad_F1\right]$ (11) $Cad _ F 1 = OC \times {k}_{Cad _ F 1}$ (12) $2 nd Metric = \left(Pwr_2M+Cad_2M\right) \times Mult _ 2$ (13) where *Pwr_2M* is a sum of 2^{nd} Metric power component data points, Pwr₂(t) is an average (e.g., an EWMA) of the power generated by the user in a time window having the second duration at time point *t* during the activity, MAP is the MAP of the user, Pwr_F2 is a second power floor, k_{Pwr_B1} is a first power boundary constant, k_{Pwr_B2} is a second power boundary constant, Pwr_C2 is a second power ceiling, k_{Pwr_C2} is a second power ceiling constant, *Cad*_2*M* is a sum of 2^{nd} Metric cadence component data points, k_{Eff} is an efficiency factor, Cad₂(t) is an average (e.g., an EWMA) of the cadence of the user in a time window having the second duration at time point *t* during the activity, Cad_F1 is a first cadence floor, OC is the optimal cadence of the user, k_{Cad_F1} is a first cadence floor constant, and Mult_2 is a second multiplier.

As shown in equation (8), the value of a 2^{nd} Metric power component data point Pwr_2M_{dpt} calculated at time *t* is only included in the sum of power component data points Pwr_2M when the power value Pwr₂(t) at time tis greater than or equal to a second power floor Pwr_F2 and less than or equal to the MAP of the user multiplied by the second power ceiling constant k_{Pwr_C2}, where the second power floor Pwr_F2 is the maximum value of the MAP of the user multiplied by the first power boundary constant k_{Pwr_B1} and the FTP of the user multiplied by the second power boundary constant k_{Pwr_B2}. As shown in equation (11), the value of a 2^{nd} Metric cadence component data point Cad_2M_{dpt} calculated at time *t* is only included in the sum of cadence component data points Cad_2M if the cadence value Cad₂(t) at time *t* is greater than or equal to the optimal cadence of the user multiplied by the first cadence floor constant k_{Cad_F1}.

In equation (9), the first power boundary constant k_{Pwr_B1} is greater than or equal to 0.8 and less than or equal to 0.93. In aspects, the first power boundary constant k_{Pwr_B1} is 0.8. The second power boundary constant k_{Pwr_B2} is greater than or equal to 1 and less than or equal to 1.1. In aspects, the second power boundary constant k_{Pwr_B2} is 1.02. In equation (10), the second power ceiling constant k_{Pwr_C2} is greater than or equal to 1.2 and less than or equal to 1.4. In aspects, the second power ceiling constant k_{Pwr_C2} is 1.4. In equation (11), the efficiency factor k_{Eff} is greater than 1 and less than or equal to 1.5. In aspects, the efficiency factor k_{Eff} is 1.15. In equation (12), the first cadence floor constant k_{Cad_F1} is greater than 1.15 and less than or equal to 1.3. In aspects, the first cadence floor constant k_{Cad_F1} is 1.2. In equation (13), the second multiplier Mult_2 is configured to scale the contributions of the suprathreshold level efforts of the user relative to the contributions of the sub-threshold and threshold level aerobic efforts, anaerobic efforts, and/or neuromuscular efforts of the user during the activity. The second multiplier Mult_2 is greater than the first multiplier Mult_1. In aspects, the second multiplier Mult_2 is greater than the first multiplier Mult_1 by a factor of 2. In aspects, the second multiplier Mult_2 is 8.

In a fifth step 150, a third metric (3^{rd} Metric) indicative of anaerobic strain experienced by the user during the activity is calculated by the processor 42. The 3^{rd} Metric quantifies the total physiological strain or load associated with anaerobic efforts by the user during the activity. The 3^{rd} Metric is a sum of 3^{rd} Metric data points multiplied by a third multiplier, with each 3^{rd} Metric data point corresponding to a single time point in the set of periodic time points during the activity. Each 3^{rd} Metric data point is a sum of 3^{rd} Metric power component and a 3^{rd} Metric anaerobic spike component. The 3^{rd} Metric power component is a sum of 3^{rd} Metric power component data points, with each power component data point corresponding to a single time point in the set of periodic time points during the activity. Each 3^{rd} Metric power component data point is derived as a function of a ratio of a power value in the third set of power values to the AC power of the user and only contributes to the 3^{rd} Metric power component when the power value in the third set of power values is within a tolerance of the AC power of the user.

The 3^{rd} Metric anaerobic spike component is a sum of 3^{rd} Metric anaerobic spike component data points, with each anaerobic spike component data point corresponding to a single time point in the set of periodic time points during the activity. Each 3^{rd} Metric anaerobic spike component data point is derived as a function of a ratio of (i) a difference between a power value in the third set of power values and a power value in the second set of power values at a given time point in the set of periodic time points during the activity and (ii) the AC power of the user. Each 3^{rd} Metric anaerobic spike component data point only contributes to the 3^{rd} Metric anaerobic spike component when the difference between the power value in the third set of power values and the power value in the second set of power values at the given time point is greater than or equal to a predetermined fraction of the AC power of the user. As such, in aspects, calculating the 3^{rd} Metric may further comprise calculating a set of power differences as a difference between a power value in the third set of power values and a power value in the second set of power values at each time point in the set of periodic time points during the activity.

In aspects, the 3^{rd} Metric is calculated according to equations (14), (15), (16), (17), (18), (19) and (20): $Pwr _ 3 M = {\sum }_{t} \left(\frac{{Pwr}_{3} \left(t\right)}{AC}\right) , \left[Pwr_F3\leq {Pwr}_{3}\left(t\right)\leq Pwr_C3\right]$ (14) $Pwr _ F 3 = AC \times {k}_{Pwr _ F 3}$ (15) $Pwr _ C 3 = AC \times {k}_{Pwr _ C 3}$ (16) $Spk _ 3 M = {\sum }_{t} \left(\frac{{Pwr}_{Spk} \left(t\right)}{AC}\right) , \left[{Pwr}_{Spk}\left(t\right)\geq Spk_F\right]$ (17) ${Pwr}_{Spk} \left(t\right) = {Pwr}_{3} \left(t\right) - {Pwr}_{2} \left(t\right)$ (18) $Spk _ F = AC \times {k}_{Spk}$ (19) $3 rd Metric = \left(Pwr_3M+Spk_3M\right) \times Mult _ 3$ (20) where *Pwr*_3*M* is a sum of 3^{rd} Metric power component data points, Pwr₃(t) is an average (e.g., an EWMA) of the power generated by the user in a time window having the third duration at time point *t* during the activity, AC is the AC power of the user, Pwr_F3 is a third power floor, k_{Pwr_F3} is a third power floor constant, Pwr_C3 is a third power ceiling, k_{Pwr_C3} is a third power ceiling constant, *Spk*_3*M* is a sum of 3^{rd} Metric anaerobic spike component data points, Spk_F is a power spike floor, k_{Spk} is a power spike constant, and Mult_3 is a third multiplier.

As shown in equation (14), the value of a 3^{rd} Metric power component data point Pwr_3M_{dpt} calculated at time *t* is only included in the sum of 3^{rd} Metric power component data points Pwr_3M when the power value Pwr₃(t) at time *t* is greater than or equal to a third power floor Pwr_F3 and less than or equal to a third power ceiling Pwr_C3. As shown in equation (17), the value of a 3^{rd} Metric anaerobic spike component data point Spk_3M_{dpt} calculated at time *t* is only included in the sum of 3^{rd} Metric anaerobic spike component data points when the difference between an average (e.g., an EWMA) of the power generated by the user in a time window having the third duration and in a time window having the second duration at time point *t* during the activity is greater than or equal to the AC power of the user multiplied by the power spike constant k_{Spk}.

In equation (15), the third power floor constant k_{Pwr_F3} is greater than or equal to 0.7 and less than or equal to 0.85. In aspects, the third power floor constant k_{Pwr_F3} is 0.75. In equation (16), the third power ceiling constant k_{Pwr_C3} is greater than or equal to 1.8 and less than or equal to 2.5. In aspects, the third power ceiling constant k_{Pwr_C3} is 2. In equation (19), the power spike constant k_{Spk} is greater than or equal to 0.2 and less than or equal to 0.3. In aspects, the power spike constant k_{Spk} is 0.25. In equation (20), the third multiplier Mult_3 is configured to scale the contributions of the anaerobic efforts of the user relative to the contributions of the sub-threshold and threshold level aerobic efforts, suprathreshold level efforts, and/or neuromuscular efforts of the user during the activity. The third multiplier Mult_3 is greater than the first multiplier Mult_1 and the second multiplier Mult_2. In aspects, the third multiplier Mult_3 is greater than the first multiplier Mult_1 by a factor of 3. In aspects, the third multiplier Mult_3 is 12.

In a sixth step 160, a fourth metric (4^{th} Metric) indicative of neuromuscular strain experienced by the user during the activity is calculated by the processor 42. The 4^{th} Metric quantifies the total physiological strain or load associated with short, high-intensity neuromuscular efforts of the user during the activity and considers the power, cadence, and torque of the user during the activity. The 4^{th} Metric is a sum of 4^{th} Metric data points multiplied by a fourth multiplier, with each 4^{th} Metric data point corresponding to a single time point in the set of periodic time points during the activity. Each 4^{th} Metric data point is a sum of a 4th Metric power component, a 4^{th} Metric cadence component, and a 4^{th} Metric torque component.

The 4^{th} Metric power component is a sum of 4^{th} Metric power component data points, with each power component data point corresponding to a single time point in the set of periodic time points during the activity. Each 4^{th} Metric power component data point is derived as a function of a ratio of a power value in the fourth set of power values to the NM power of the user and only contributes to the 4^{th} Metric power component when the power value in the fourth set of power values is greater than a predetermined fraction of the NM power of the user.

The 4^{th} Metric cadence component is a sum of 4^{th} Metric cadence component data points, where a 4^{th} Metric cadence component data point is derived as a function of a ratio of a power value in the fourth set of power values to the NM power of the user raised to the power of a second exponent and contributes to the 4^{th} Metric cadence component when a cadence value in the second set of cadence values is greater than the optimal cadence of the user.

The 4^{th} Metric torque component is a sum of 4^{th} Metric torque component data points, where each 4^{th} Metric torque component data point is derived as a function of a ratio of a power value in the fourth set of power values to the NM power of the user raised to the power of the second exponent and contributes to the 4^{th} Metric torque component when (i) a torque value in the set of torque values at a given time point in the set of periodic time points is greater than a threshold torque of the user and (ii) a cadence value in the second set of cadence values at the given time point is greater than the optimal cadence of the user.

In aspects, the 4^{th} Metric is calculated according to equations (21), (22), (23), (24), (25), (26), (27), and (28): $Pwr _ 4 M = {\sum }_{t} \left(\frac{{Pwr}_{4} \left(t\right)}{NM}\right) , \left[Pwr_F4\leq {Pwr}_{4}\left(t\right)\right]$ (21) $Pwr _ F 4 = NM \times {k}_{Pwr _ F 4}$ (22) $Cad _ 4 M = {{\sum }_{t} \left(\frac{{Pwr}_{4} \left(t\right)}{NM}\right)}^{Exp _ 2} , \left[{Cad}_{4}\left(t\right)\geq Cad_F2\right]$ (23) $Cad _ F 2 = OC \times {k}_{Cad _ F 2}$ (24) $Trq _ 4 M = {{\sum }_{t} \left(\frac{{Pwr}_{4} \left(t\right)}{NM}\right)}^{Exp _ 2} , \left[\begin{matrix}{Trq}_{4} \left(t\right) \geq Trq _ F and {Cad}_{4} \left(t\right) \\ \leq Cad _ C 2\end{matrix}\right]$ (25) $Trq _ F = TT \times {k}_{Trq _ F}$ (26) $Cad _ C 2 = OC \times {k}_{Cad _ C 2}$ (27) $4 th Metric = \left(Pwr_4M+Cad_4M+Trq_4M\right) \times Mult _ 4$ (28) where *Pwr*_4*M* is a sum of 4^{th} Metric power component data points, Pwr₄(t) is an average (e.g., an EWMA) of the power generated by the user in a time window having the fourth duration at time point *t* during the activity, NM is the NM power of the user, Pwr_F4 is a fourth power floor, k_{Pwr_F4} is a fourth power floor constant, Exp_2 is a second exponent, *Cad*_4*M* is a sum of 4^{th} Metric cadence component data points, Cad₄(t) is an average (e.g., an EWMA) of the cadence of the user in a time window having the fourth duration at time point *t* during the activity, OC is the optimal cadence of the user, Cad_F2 is a second cadence floor, k_{Cad_F2} is a second cadence floor constant, *Trq*_4*M* is a sum of 4^{th} Metric torque component data points, Trq₄(t) is an average (e.g., an EWMA) of the torque generated by the user in a time window having the fourth duration at time point *t* during the activity, Trq_F is a torque floor, TT is the threshold torque of the user, k_{Trq_F} is a torque floor constant, Cad_C2 is a second cadence ceiling, k_{Cad_C2} is a second cadence ceiling constant, and Mult_4 is a fourth multiplier.

As shown in equation (21), the value of a 4^{th} Metric power component data point Pwr_4M_{dpt} calculated at time *t* is only included in the sum of 4^{th} Metric power component data points Pwr_4M when the power value Pwr₄(t) at time *t* is greater than or equal to a fourth power floor Pwr_F4. As shown in equation (23), the value of a 4^{th} Metric cadence component data point Cad_4M_{dpt} calculated at time *t* is only included in the sum of 4^{th} Metric cadence component data points Cad_4M when the cadence value Cad₄(t) at time *t* is greater than or equal to a second cadence floor Cad_F2. As shown in equation (25), the value of a 4^{th} Metric torque component data point Trq_4M_{dpt} calculated at time *t* is only included in the sum of 4^{th} Metric torque component data points Trq_4M when the torque value Trq₄(t) at time *t* is greater than or equal to a torque floor Trq_F and the cadence value Cad₄(t) at time *t* is less than or equal to a second cadence ceiling Cad_C2.

In equation (22), the fourth power floor constant k_{Pwr_F4} is greater than or equal to 0.65 and less than or equal to 0.8. In aspects, the fourth power floor constant k_{Pwr_F3} is 0.75. In equation (23), the second exponent Exp_2 is greater than 1. In aspects, the second exponent Exp_2 is 2. In equation (24), the second cadence floor constant k_{Cad_F2} is less than 1. In aspects, the second cadence floor constant k_{Cad_F2} is 0.75. In equation (26), the torque floor constant k_{Trq_F} is greater than or equal to 2 and less than or equal to 2.5. In aspects, the torque floor constant k_{Trq_F} is 2.25. In equation (27), the second cadence ceiling constant k_{Cad_C2} is greater than or equal to 1.2 and less than or equal to 1.4. In aspects, the second cadence ceiling constant k_{Cad_C2} is 1.25. In equation (28), the fourth multiplier Mult_4 is configured to scale the contributions of the neuromuscular efforts of the user relative to the contributions of the sub-threshold and threshold level aerobic efforts, suprathreshold level efforts, and/or anaerobic efforts of the user during the activity. The fourth multiplier Mult_4 is greater than the first multiplier Mult_1, the second multiplier Mult_2, and the third multiplier Mult_3. In aspects, the fourth multiplier Mult_4 is greater than the first multiplier Mult_1 by a factor of greater than 8 and is greater than the third multiplier Mult_3 by a factor of greater than 2. In aspects, the fourth multiplier Mult_4 is 35.

In a seventh step 170, to ensure comparability of the 1^{st} Metric, the 2^{nd} Metric, the 3^{rd} Metric, and the 4^{th} Metric across different users and different activities, the 1^{st} Metric, the 2^{nd} Metric, the 3^{rd} Metric, and the 4^{th} Metric are normalized to base 100 by dividing each metric by a metric base division (MBD) factor. The MBD factor is derived as a function of the duration between each time point in the set of periodic time points during the activity. For example, in aspects where the duration between each time point in the set of periodic time points is 1 second, the MBD factor is 144.

In an eighth step 180, a multicomponent Intensity Density (ID) Score is calculated by the processor 42. As shown in equation (29), the ID Score is a sum of the 1^{st} Metric, the 2^{nd} Metric, the 3^{rd} Metric, and the 4^{th} Metric.$ID Score = 1 st Metric + 2 nd Metric + 3 rd Metric + 4 th Metric$ (29)

In a nineth step 190, the ID Score is provided to the user, for example, by displaying the ID Score to the user via the output device 38. Additionally or alternatively, in the nineth step 190, the 1^{st} Metric, the 2^{nd} Metric, the 3^{rd} Metric, and/or the 4^{th} Metric may be provided to the user. In aspects, in the nineth step 190, indicia of the 1^{st} Metric, the 2^{nd} Metric, the 3^{rd} Metric, and/or the 4^{th} Metric may be provided to the user. For example, in the nineth step 190, the ID Score may be provided to the user along with an indication of the portion of the ID Score (e.g., the percentage of the ID Score) attributed to by the 1^{st} Metric, the 2^{nd} Metric, the 3^{rd} Metric, and/or the 4^{th} Metric.

In aspects, the 1^{st} Metric, the 2^{nd} Metric, the 3^{rd} Metric, the 4^{th} Metric, and/or the ID Score may be calculated by the computer 18 prior to completion of the activity. For example, in aspects, the 1^{st} Metric, the 2^{nd} Metric, the 3^{rd} Metric, the 4^{th} Metric, and/or the ID Score may be calculated continuously or at particular times during the activity. In such case, the value of the 1^{st} Metric, the 2^{nd} Metric, the 3^{rd} Metric, the 4^{th} Metric, and/or the ID Score accumulated up to a particular time during the activity may be provided to the user. Additionally or alternatively, values of the 1^{st}, 2^{nd}, 3^{rd}, and/or 4^{th} Metric data points may be provided to the user at one or more time points during the activity. In aspects, a sum of the 1^{st}, 2^{nd}, 3^{rd}, and 4^{th} Metric data points may be provided to the user at one or more time points during the activity and may represent an Intensity of the activity at that point during the activity.

### EXAMPLES

### Power Profile Assessment

The FTP, MAP, AC power, and NM power of a user-collectively referred to herein as the Power Profile of the user-may be assessed by having the user perform a 1-hour Cycling Test that requires the user to sequentially perform four structured tests separated by periods of recovery while measuring and recording the power and cadence of the user during the tests. A description of the 1-Hour Cycling Test and the four structured tests is shown in **Table 1** below. Proper pacing during each of the tests is critical to getting the most accurate Power Profile results.

**Table 1. 1-Hour Cycling Test**

| | |
|---|---|
| Warm Up: ~15 minutes | |
| **Test #1**: Neuromuscular (NM) Power Assessment | |
| | Total Duration: ~ 4 minutes |
| | Number of Sprints: 2 |
| | Sprint Duration: 5-seconds |
| | Rest between Sprints: ~ 4 minutes |
| Recovery #1: ~ 3 minutes | |
| **Test #2:** Maximal Aerobic Power (MAP) Assessment | |
| | Total Duration: ~ 5 minutes |
| Recovery #2: ~ 5 minutes | |
| **Test #3:** Functional Threshold Power (FTP) Assessment | |
| | Total Duration: ~ 20 minutes |
| Recovery: ~ 5 minutes | |
| **Test #4:** Anaerobic Capacity (AC) Power Assessment | |
| | Total Duration: ~ 1 minute |
| Cool Down: ~ 4 minutes | |

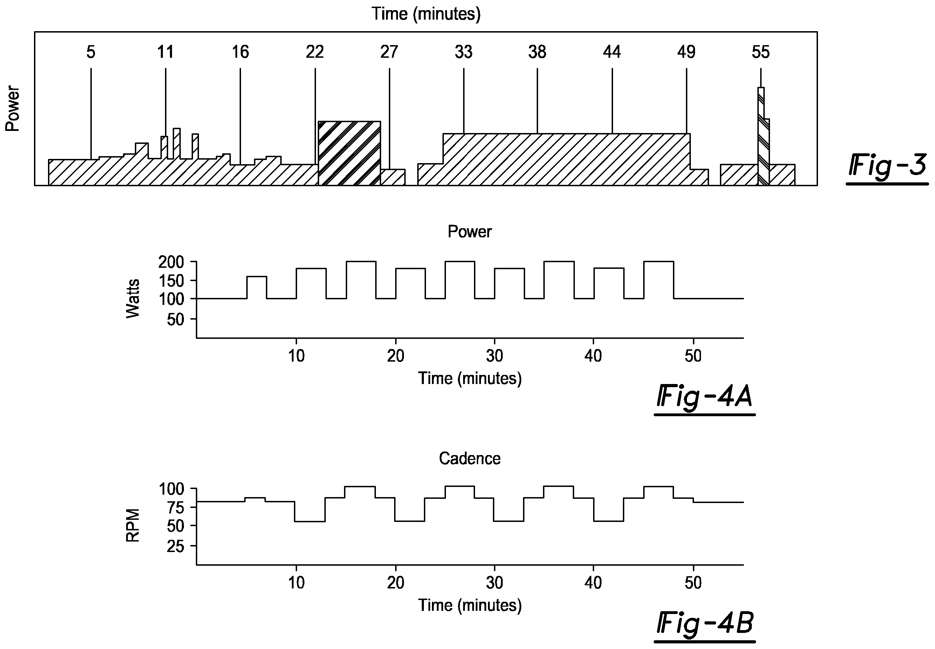

**FIG. 3** depicts an example power profile of a user during the 1-Hour Cycling Test.

The easy, progressive Warm Up features a couple of moderate-intensity efforts to get the user's legs and cardiovascular system ready for the tests.

Test #1 measures the user's NM Power, i.e., the maximal 5-second sustained power of the user. The key to getting the highest numbers possible is to find the perfect balance between resistance and cadence, force and velocity. Power is affected by both. Spin too fast and you won't generate sufficient force. Grind it out and you won't get sufficient velocity. If you're an experienced rider, aim for a cadence in the 100-110 rpm range. If you're new to cycling or don't have that many miles in your legs, aim for 90-100 rpm. After completion of the 1-Hour Cycling Test, the user's NM Power is calculated based on the user's best 5-second power across both sprints.

Test #2 measures the user's MAP, the 5-minute maximal power of the user. Prior to performing Test #2, the user may be instructed that the key to Test #2 is being smart about pacing. As far as cadence goes, the user may be instructed to pedal at a slightly higher cadence than what they would consider their "optimal cadence."

Test #3 is used to determine the user's FTP and is calculated based on the maximum sustained power of the user during the 20-minute effort of Test #3 and, to be accurate, must be performed after Test #1 and Test #2. After Tests #1 and #2, the user is not going to feel fresh, so they will not be able to generate the same amount of sustained power during Test #3 as they would in other 20-minute FTP tests. Because of this, unlike other FTP tests, the average power generated by the user during the full 20-minutes of Test #3 may be used to calculate the user's FTP, instead of calculating the user's FTP as 95% of the average power generated by the user during a 20-minute during when the user is fresh. To get accurate results from Test #3, it is preferable for the user to have gone all-out in Test #2. Sandbagging any part of the 1-Hour Cycling Test may result in inaccurate values for the Power Profile of the user.

Prior to Test #3, the user may be instructed that their performance in Test #2 may give them a good idea of how they should pace Test #3. For example, the first 10 minutes of Test #3, the user may be instructed to aim for 80% of their average power during Test #2. 10 minutes into Test #3, the user may be instructed to increase or decrease their power output depending upon how they are feeling. During Test #3, the user may be instructed to settle into whatever cadence they consider optimal. The optimal cadence for newer riders may be somewhere around 75-80 rpm, and more experienced riders should aim for an average cadence of 95 rpm during Test #3. Again, the user may be instructed to focus on finding the perfect balance between resistance and cadence to achieve maximal sustained power output.

Test #4 measures the user's AC Power and is assessed by asking the user to perform a 1-minute all-out effort. It is assumed that, in Test #4, the user will be fatigued, and thus Test #4 is designed to assess how much power the user can generate for 1 minute when fatigued. Prior to Test #4, the user may be instructed that the best pacing strategy in Test #4 is to hit roughly 2x your average power during Test #2 for the first 10-15 seconds of Test #4 and then just try and hold on for dear life. Unlike Test #2 and #3, the power output of a user during Test #4 may look like a slight peak and fade. Some users will get out of the saddle for the first 15-20 seconds of Test #4, sit down for another 20-30 seconds, and then spend the last 15-10 seconds out of the saddle to really get everything out. Since Test #4 is a shorter effort, the user may be instructed that a higher than "optimal cadence" is recommended but that, due to fatigue, it may be difficult to achieve a very high cadence. The user may be reminded that that the lower their cadence, the more force they have to put out on each pedal stroke to generate the same amount of power. The cadence of the user during Test #4 will likely vary as they try to find a gear that feels "good."

### Cycling Activity 1: Cadence & Intensity Density

A first user performed a cycling activity, Cycling Activity 1, and the power and cadence of the user were recorded during the activity as a function of time. The following performance metrics were predetermined for the first user: FTP=200 W; MAP=250 W; AC power=350 W; NM power=800; OC=80 rpm; and TT=23.87 N·m. The power and cadence of the user during the activity respectively expressed as a percentage of the FTP and OC of the user are shown in **Table 2** below. As shown, each interval during the activity includes a low-cadence 3-minute "effort" at 90% of FTP and 68% of OC and a high-cadence 3-minute "effort" at 100% of FTP and 120% of OC.

**Table 2. Cycling Activity 1**

| | | |
|---|---|---|
| Warm Up: 10 minutes close to 100% OC | | |
| | 5 minutes at 50% FTP | |
| | 2 minutes at 80% FTP | |
| | 3 minutes at 50% FTP | |
| Main Effort: 38 minutes | | |
| | Number of Intervals: 4 | |
| | Interval: | 3 minutes at 90% FTP at 55 rpm (68% of OC) |
| | | 2 minutes at 50% FTP at 80 rpm (100% OC) |
| | | 3 minutes at 100% FTP at 100 rpm (120% OC) |
| | Rest between Intervals: 2 minutes at 50% FTP at 80 rpm (100% OC) | |
| Cool Down: 7 minutes at 50% FTP close to 100% OC | | |

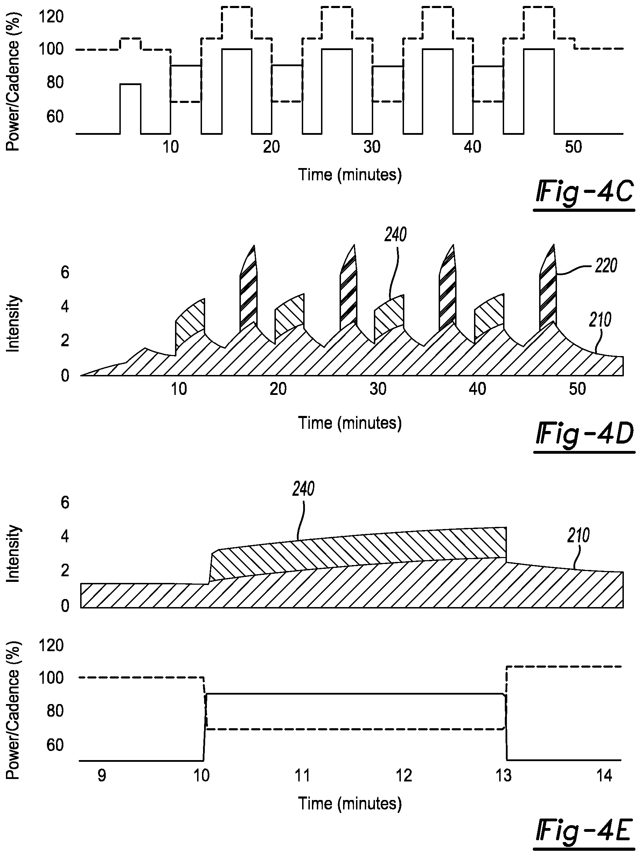

**FIGS. 4A and 4B** respectively depict profiles of the power and cadence of the user during Cycling Activity 1. **FIG. 4C** depicts a plot of the power (solid line) and cadence (dashed line) of the user during the activity respectively expressed as a percentage of the user's FTP and OC.

**Table 3** below shows the values of the 1^{st} Metric, the 2nd Metric, the 3^{rd} Metric, the 4^{th} Metric, the cumulative ID Score, and the Training Stress Score (TSS) of the activity.

**Table 3.**

| | Total | % of ID Score |
|---|---|---|
| Intensity Density Score | 64.7 | 100 |
| 1^{st} Metric | 45.2 | 70 |
| 2^{nd} Metric | 10.9 | 17 |
| 3^{rd} Metric | 0 | 0 |
| 4^{th} Metric | 8.7 | 13 |
| TSS | 57.6 | -- |

**FIG. 4D** depicts a stacked area plot of the 1^{st} Metric (210), the 2^{nd} Metric (220), and the 4^{th} Metric (240) during the activity. Throughout the duration of Cycling Activity 1, the value of the 3^{rd} Metric was zero.

Because the power of the user for the entire duration of Cycling Activity 1 is at or below 100% of the user's FTP, methods of quantifying the physical load experienced by the user during the activity that only consider the power output of the user during the activity relative to their FTP (e.g., TSS) do not provide a comprehensive assessment of the physical load experienced by the user because they do not consider how the cadence of the user impacts the type and degree of metabolic strain experienced by the user. In addition, such methods are not individualized because they do not consider the cadence of the user during the activity relative to the OC of the user.

The Effect of Cadence During Cycling Activity 1 Explained: The shifts in cadence during the activity cause a shift in the physiological demands on the user. In the case of the low-cadence efforts, despite the power output of the user being below 100% of FTP, the torque exerted by the user 131% of their Threshold Torque. Conversely, the higher-cadence efforts reduce the user's efficiency and are estimated to increase the oxygen demand of these efforts to about 115% of what the oxygen demand would be at the same power output if the user was cycling at their optimal cadence.

In other words, the increases and decreases in the cadence of the user relative to their OC change the types of strain experienced by the user during each part of the activity. During the low-cadence efforts, when the user is pedaling at a cadence below their OC, even though the power is below their FTP (and therefore indicates sub-threshold or threshold level aerobic strain accounted for by the 1^{st} Metric, 210), each pedal stroke requires about 30% more force than what the user would apply at their TT, which imparts neuromuscular strain on the user and is accounted for in the 4^{th} Metric (240). On the flip side, during the high-cadence efforts, when the user pedals at a cadence above their OC, the user's muscles become less efficient and the user ends up using about 115% more oxygen than they would if they were pedaling at their OC, which imparts anaerobic strain on the user and is accounted for in the 2^{nd} Metric (220).

As shown in **FIG. 4D**, for the entire duration of the activity the user is experiencing and accumulating sub-threshold or threshold level aerobic strain, which is quantified by the 1^{st} Metric (210). This is because at no point during the activity does the power output of the user exceed 100% of their FTP.

Referring now to **FIG. 4E**, which depicts an enlarged view of a portion of the plot of **FIG. 4C** and a portion of the stacked area plot of **FIG. 4D** covering one of the low-cadence efforts, we see that the user begins to experience and accumulate neuromuscular strain quantified by the 4^{th} Metric (240) almost immediately after the low-cadence effort begins, which is due to the fact that the torque generated by the user during the low-cadence effort is 131% of the user's threshold torque and because the 4^{th} Metric (240) is derived as a function of an exponentially weighted moving average (EWMA) of the torque of the user in a time window having a relatively short duration, i.e., in this case, 5 seconds.

In addition, as the duration of the low-cadence effort increases, the rate the user is accumulating sub-threshold or threshold level aerobic strain increases due to the fact that the 1^{st} Metric (210) is derived as a function of an EWMA of the power of the user in a time window having a relatively long duration, i.e., in this case, 5 minutes. As such, as the duration of the low-cadence effort increases, so does the power value used in the calculation of the 1^{st} Metric (210).

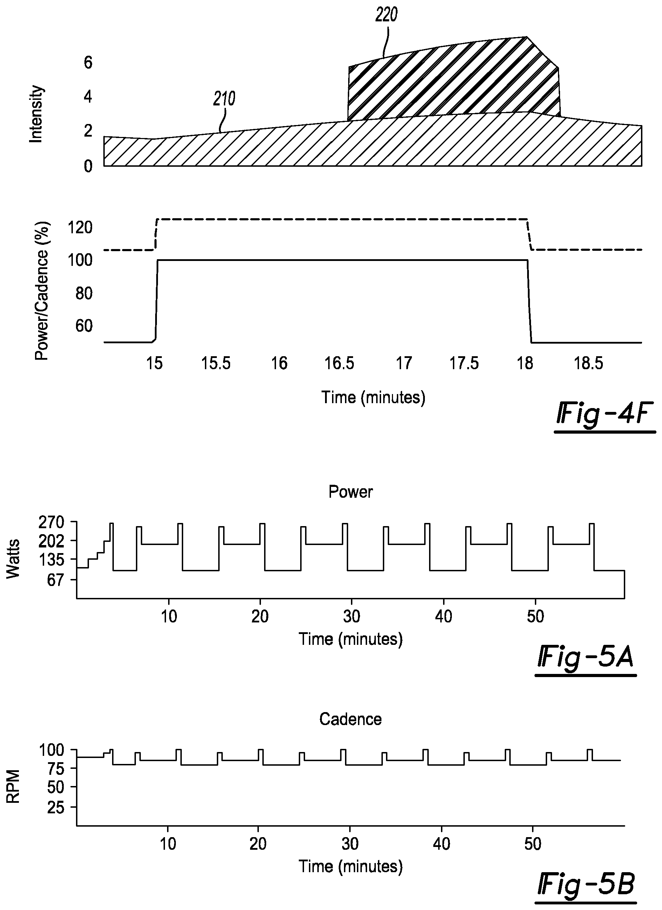

Referring now to **FIG. 4F**, which depicts an enlarged view of a portion of the plot of **FIG. 4C** and a portion of the stacked area plot of **FIG. 4D** covering one of the high-cadence efforts, we see that the user only begins to experience and accumulate suprathreshold level strain quantified by the 2^{nd} Metric (220) in the second half of the effort. Even though the cadence of the user immediately increases at the start of the high-cadence effort, there is a lag in the ability of the metabolic system of the user to adapt to the new type of strain. This lag in the metabolic response of the user is accounted for in the 2^{nd} Metric (220), which is derived as a function of an EWMA of the power and cadence of the user in a time window having a relatively long duration, i.e., in this case, 2 minutes, which means that the value of the 2^{nd} Metric (220) will only increase after increases in the power and/or cadence of the user have persisted. For the same reason, even after completion of a 3-minute high-cadence effort, the user continues to experience and accumulate suprathreshold level strain quantified by the 2^{nd} Metric (220) and is in keeping with the response characteristics of certain metabolic processes in the body, which may gradually taper off after the physical effort that initiated the metabolic process has ended.

In sum, quantifying the physiological strain experienced during an activity based on a combination of metrics that are each derived as a function of the average power, cadence, and/or torque of the user in a time window having a different duration allows for a more granular and precise assessment of the physiological strain experienced by the user. In addition, because the metrics used to quantify physiological strain are derived as a function of the user's FTP, as well as the user's MAP, AC power, NM power, OC, and TT, the ID Score provides a more individualized assessment of the actual physiological strain experienced by the user during the activity.

### Cycling Activity 2: Power Surges & Intensity Density

The same user as in Cycling Activity 1 performed another cycling activity, Cycling Activity 2, and the power and cadence of the user were recorded during the activity as a function of time. The power and cadence of the user during the interval portion of the activity respectively expressed as a percentage of the FTP and OC of the user are shown in **Table 4** below. As shown, each interval during the activity includes two 30-second high-power and high-cadence "surges" separated by a 4-minute steady-state effort.

**Table 4. Cycling Activity 2**

| | |
|---|---|
| Number of Intervals: 6 | |
| Interval: | 30 seconds at 125% FTP at 120% of OC |
| | 4 minutes at 95% FTP at 105% OC |
| | 30 seconds at 130% FTP at 125% OC |
| Rest between Intervals: 4 minutes at 50% FTP at 80 rpm (100% OC) | |

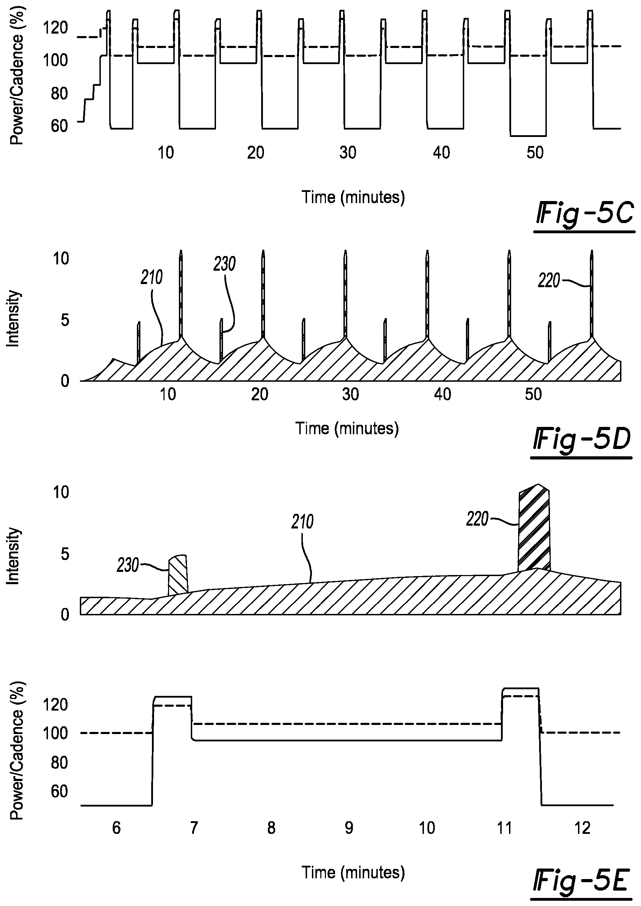

**FIGS. 5A and 5B** respectively depict profiles of the power and cadence of the user during Cycling Activity 2. **FIG. 5C** depicts a plot of the power (solid line) and cadence (dashed line) of the user during the activity respectively expressed as a percentage of the user's FTP and OC.

**Table** 5 below shows the values of the 1^{st} Metric, the 2^{nd} Metric, the 3^{rd} Metric, the 4^{th} Metric, the ID Score, and the Training Stress Score (TSS) of the activity.

**Table 5.**

| | Total | % of ID Score |
|---|---|---|
| Intensity Density Score | 67.2 | 100 |
| 1^{st} Metric | 58 | 86 |
| 2^{nd} Metric | 6.7 | 10 |
| 3^{rd} Metric | 2.5 | 4 |
| 4^{th} Metric | 0 | 0 |
| TSS | 76.6 | -- |

**FIG. 5D** depicts a stacked area plot of the 1^{st} Metric (210), the 2^{nd} Metric (220), and the 3^{rd} Metric (230) during the activity. Throughout the duration of Cycling Activity 2, the value of the 4^{th} Metric was zero.

As shown in **FIG. 5D**, for the entire duration of the activity the user is experiencing and accumulating sub-threshold or threshold level aerobic strain, which is quantified by the 1^{st} Metric (210). This is because the 1^{st} Metric (210) is derived as a function of an EWMA of the power of the user in a time window having the longest duration of all four metrics, in this case 5 minutes, and at no point during the activity does the EWMA of the power of the user exceed 100% of their FTP.

In addition, during each 4-minute steady-state effort, the rate the 1^{st} Metric (210) is accumulating increases, which also results from the fact that the 1^{st} Metric (210) is derived as a function of an EWMA of the power of the user in a time window having a relatively long duration, i.e., 5 minutes, which means that, as the duration of the effort increases, so does the power value used in the calculation of the 1^{st} Metric (210). This result aligns with the perceived exertion of the user because, as the duration of the steady-state effort increases, each additional second of effort will generally feel "harder" to the user than the effort at the beginning, even though the power output of the user remains constant during the steady-state effort.

Referring now to **FIG. 5E**, which depicts an enlarged view of a portion of the plot of **FIG. 5C** and a portion of the stacked area plot of **FIG. 5D** covering one of the intervals. Looking at one of the first high-power and high-cadence surges at the start of the interval, we see that the user begins to experience and accumulate anaerobic strain quantified by the 3^{rd} Metric (230) roughly 15 seconds after the start of the interval and stops accumulating anaerobic strain as soon as the surge is over. This is because the 3^{rd} Metric (230) is derived as a function of an EWMA of the power of the user in a time window having a relatively short duration, in this case 15 seconds, and because the 3^{rd} Metric (230) includes an anaerobic spike component, which is formulated to increase the value of the 3^{rd} Metric (230) in response to spikes in the power output of the user that are greater than or equal to a predetermined fraction of the AC power of the user. This result aligns with the expected metabolic response of the user, which, because the heart rate and breathing rate of the user are expected to be relatively low at the start of the interval, to meet the spike in power output required during the first surge, is likely to rely on anaerobic metabolic pathways.

By comparison, as shown best in **FIG. 5E**, looking at the second high-power and high-cadence surge at the end of the interval, we see that the user begins to experience and accumulate suprathreshold level strain quantified by the 2^{nd} Metric (220) roughly 15 seconds after the start of the second surge. This is because the 2^{nd} Metric (220) is derived as a function of a ratio of an EWMA of the power of the user in a time window having a relatively long duration, in this case 2 minutes, and the MAP of the user. Because the second surge comes at the end of a 4-minute steady-state effort where the power output of the user was near the FTP of the user, when the power output of the user exceeds the FTP of the user and approaches the MAP of the user at the start of the second surge, the EWMA of the power of the user over the previous 2 minutes will rapidly approach or exceed the MAP of the user and the equation used to calculate the 2^{nd} Metric (220) with return a positive value. This result aligns with the expected metabolic response of the user, which, because the heart rate and breathing rate of the user are expected to be relatively high at the end of the interval, increases in the physiological demands on the user are expected to push the physiological strain of the user to suprathreshold levels.

### Cycling Activity 3: Comparison of Users with Different Performance Metrics

A second user performed the same cycling activity as in Cycling Activity 2 and the power and cadence of the user were recorded during the activity as a function of time. The following performance metrics were predetermined for the second user: FTP=200 W; MAP=250 W; AC power=325 W; NM power=800; OC=80 rpm; and TT=23.87 N·m. As shown, in comparison to the first user, the second user had relatively low AC power.

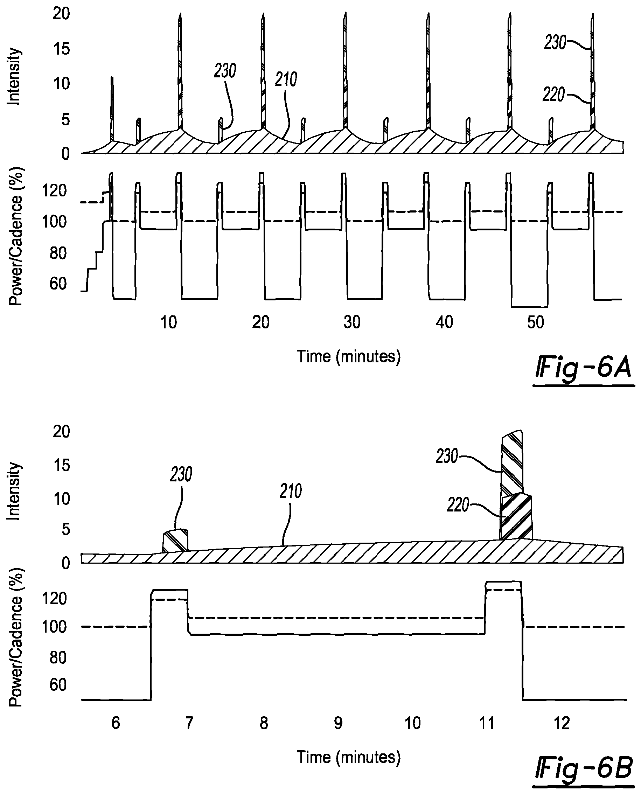

**FIG. 6A** depicts a stacked area plot of the 1^{st} Metric (210), the 2^{nd} Metric (220), and the 3^{rd} Metric (230) during Cycling Activity 3, and a plot of the power (solid line) and cadence (dashed line) of the second low-AC power user during the activity respectively expressed as a percentage of the second user's FTP and OC. **FIG. 6B** depicts an enlarged view of a portion of the plot of **FIG. 6A** covering one of the intervals during Cycling Activity 3.

**Table 6** below shows the values of the 1^{st} Metric, the 2^{nd} Metric, the 3^{rd} Metric, the 4^{th} Metric, the ID Score, and the Training Stress Score (TSS) of Cycling Activity 3.

**Table 6.**

| | Total | % of ID Score |
|---|---|---|
| Intensity Density Score | 75.4 | 100 |
| 1^{st} Metric | 58 | 77 |
| 2^{nd} Metric | 6.7 | 9 |
| 3^{rd} Metric | 10.7 | 14 |
| 4^{th} Metric | 0 | 0 |
| TSS | 76.6 | -- |

As shown in **FIGS. 5D** **and** **6A**, for the entire duration of Cycling Activity 2 and Cycling Activity 3 both the first and second user experience and accumulate the same amount of sub-threshold or threshold level aerobic strain (quantified by the 1^{st} Metric, 210) and the same amount of suprathreshold level strain (quantified by the 2^{nd} Metric, 220) because the first user and the second user have the same FTP and the same MAP.

As best shown in **FIG. 6B**, looking at the second high-power and high-cadence surge at the end of the interval, we see that, unlike the first user in Cycling Activity 2, the second user with a relatively low AC power begins to experience and accumulate suprathreshold level strain quantified by the 2^{nd} Metric (220) as well as anaerobic strain quantified by the 3^{rd} Metric (230) after the start of the second surge. This is due to the fact that the AC power of the second user is lower than that of the first user and thus, even though the power output of the first and second user is the same and represents the same % of their FTP, the relatively low AC power of the second user means that the EWMA of the power of the user during the second 30-second surge falls within a tolerance of the AC power of the second user (is between the third power floor Pwr_F3 and the third power ceiling Pwr_C3 in equation (14)), and thus contributes to 3^{rd} Metric (230). This result aligns with the expected metabolic response of the second user, which, because of the second user's relatively low AC power, the second 30-second power surge at the end of the interval is likely to require the second user to tap into their anaerobic metabolic pathways to achieve the power outputs required. Notably, during the second 30-second power surge at the end of the interval, the second user continues to experience the same amount of aerobic strain (quantified by the 2^{nd} Metric, 220) as the first user because both users have the same MAP.

In addition, as shown in **Tables 5 and 6**, using TSS to quantify the loads experienced by the users in Cycling Activity 2 and Cycling Activity 3 results in the same quantity because such method only considers the power output of the user during the activity relative to their FTP and does not consider other performance metrics of the user, such as MAP in this scenario.

Unless specifically stated otherwise as apparent from the above discussion, it is appreciated that throughout the description, discussions utilizing terms such as "processing" or "computing" or "calculating" or "determining" or "displaying" or the like, refer to the action and processes of the processor 42 of the computer 18, or similar electronic computing device, that manipulates and transforms data represented as physical (electronic) quantities within the memory 40 of the computer 18 or registers or other such information storage, transmission or display devices. Certain aspects of the described techniques include process steps and instructions described herein in the form of an algorithm. It should be noted that the described process steps and instructions could be embodied in software, firmware or hardware, and when embodied in software, could be downloaded to reside on and be operated from different platforms used by real time network operating systems.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

Throughout this disclosure, the numerical values represent approximate measures or limits to ranges and encompass minor deviations from the given values and embodiments, having about the value mentioned as well as those having exactly the value mentioned. Other than the working examples provided at the end of the detailed description, all numerical values of parameters (e.g., of quantities or conditions) in this specification are to be understood as being modified in all instances by the term "about" whether or not "about" actually appears before the numerical value. Numerical values of parameters in the appended claims are to be understood as being modified by the term "about" only when such term appears before the numerical value. "About" indicates that the stated numerical value allows some slight imprecision (with some approach to exactness in the value; approximately or reasonably close to the value; nearly). If the imprecision provided by "about" is not otherwise understood in the art with this ordinary meaning, then "about" as used herein indicates at least variations that may arise from ordinary methods of measuring and using such parameters. For example, "about" may comprise a variation of less than or equal to 10%, optionally less than or equal to 5%, optionally less than or equal to 4%, optionally less than or equal to 3%, optionally less than or equal to 2%, optionally less than or equal to 1%, optionally less than or equal to 0.5%, and in certain aspects, optionally less than or equal to 0.1%. In addition, disclosure of ranges includes disclosure of all values and further divided ranges within the entire range, including endpoints and sub-ranges given for the ranges.

## Claims

1. A computer-implemented method for quantitation of physiological strain experienced by a user during a cycling activity, the method comprising:
receiving, by a processor of a computer, measurements of power exerted by the user during the activity;
calculating, by the processor of the computer, a first metric indicative of sub-threshold and threshold level aerobic strain experienced by the user during the activity;
calculating, by the processor of the computer, a second metric indicative of suprathreshold level strain experienced by the user during the activity;
calculating, by the processor of the computer, a third metric indicative of anaerobic strain experienced by the user during the activity;
calculating, by the processor of the computer, a fourth metric indicative of neuromuscular strain experienced by the user during the activity, where the first, second, third, and fourth metrics are each derived as a function of the measurements of the power exerted by the user during the activity; and
displaying, by a display of the computer, indicia of the first metric, the second metric, the third metric, and the fourth metric.

2. The method of claim 1, further comprising:
storing, in memory of the computer, a functional threshold power (FTP) of the user, a maximal aerobic power (MAP) of the user, an anaerobic capacity (AC) power of the user, and a neuromuscular (NM) power of the user,
wherein the first metric is further derived as a function of the FTP of the user, the second metric is further derived as a function of the MAP of the user, the third metric is further derived as a function of the AC power of the user, and the fourth metric is further derived as a function of the NM power of the user,
optionally wherein the FTP of the user is the maximum steady-state aerobic power that can be maintained by the user for a duration of about 60 minutes, the MAP of the user is the maximum power that can be maintained by the user for a duration of about 5 minutes, the AC power of the user is the maximum power that can be maintained by the user for a duration of about 1 minute when fatigued, and the NM power of the user is the maximum power that can be maintained by the user for a duration of about 5 seconds.

3. The method of claim 1, further comprising:
calculating, by the processor of the computer, a first set of power values by averaging the measurements of the power exerted by the user during the activity in a time window having a first duration at each time point in a set of periodic time points during the activity;
calculating, by the processor of the computer, a second set of power values by averaging the measurements of the power exerted by the user during the activity in a time window having a second duration at each time point in the set of periodic time points during the activity;
calculating, by the processor of the computer, a third set of power values by averaging the measurements of the power exerted by the user during the activity in a time window having a third duration at each time point in the set of periodic time points during the activity; and
calculating, by the processor of the computer, a fourth set of power values by averaging the measurements of the power exerted by the user during the activity in a time window having a fourth duration at each time point in the set of periodic time points during the activity,
wherein the first duration is greater than the second duration, the second duration is greater than the third duration, and the third duration is greater than the fourth duration.

4. The method of claim 3, further comprising:
measuring, by a power meter, the power exerted by the user during the activity.

5. The method of claim 3, wherein:
the first duration is 4 minutes to 6 minutes, the second duration is 1 minute to 3 minutes, the third duration is 10 seconds to 30 seconds, and the fourth duration is one second to less than 10 seconds;
and/or
the first metric is derived as a function of the first set of power values, the second metric is derived as a function of the second set of power values, the third metric is derived as a function of the third set of power values, and the fourth metric is derived as a function of the fourth set of power values.

6. The method of claim 3, wherein calculating the first metric comprises:
calculating a sum of first metric data points, where a first metric data point is derived as a function of a ratio of a power value in the first set of power values to a functional threshold power (FTP) of the user and contributes to the first metric when the power value in the first set of power values is within a predefined tolerance of the FTP of the user, where the FTP of the user is the maximum steady-state aerobic power that can be maintained by the user for a duration of about 60 minutes.

7. The method of claim 6, wherein calculating the third metric comprises:
calculating a set of power differences as a difference between a power value in the third set of power values and a power value in the second set of power values at each time point in the set of periodic time points during the activity; and
calculating a sum of a third metric power component and an anaerobic spike component,
wherein the third metric power component is a sum of third metric power component data points, where a third metric power component data point is derived as a function of a ratio of a power value in the third set of power values to an anaerobic capacity (AC) power of the user and contributes to the third metric power component when the power value in the third set of power values is within a tolerance of the AC power of the user,
wherein the anaerobic spike component is a sum of anaerobic spike component data points, where an anaerobic spike component data point is derived as a function of a ratio of a power difference in the set of power differences to the AC power of the user and contributes to the anaerobic spike component when the power difference in the set of power differences is greater than or equal to a predetermined fraction of the AC power of the user, and
wherein the AC power of the user is the maximum power that can be maintained by the user for a duration of about 1 minute when fatigued.

8. The method of claim 7, further comprising:
receiving, by the processor of the computer, measurements of a cadence of the user during the activity;
calculating, by the processor of the computer, a first set of cadence values by averaging measurements of the cadence of the user during the activity in a time window having the second duration at each time point in the set of periodic time points during the activity; and
calculating, by the processor of the computer, a second set of cadence values by averaging measurements of the cadence of the user during the activity in a time window having the fourth duration at each time point in the set of periodic time points during the activity,
wherein the second metric is further derived as a function of the first set of cadence values, the fourth metric is further derived as a function of the second set of cadence values, and
wherein the second metric and the fourth metric are each further derived as a function of a predefined optimal cadence of the user.

9. The method of claim 8, further comprising:
measuring, by a cadence sensor, the cadence of the user during the activity;
and/or
receiving, by a user interface of the computer, an input value for the predefined optimal cadence of the user.

10. The method of claim 8, wherein calculating the second metric comprises:
calculating a sum of a second metric power component and a second metric cadence component,
wherein the second metric power component is a sum of second metric power component data points, where a second metric power component data point is derived as a function of a ratio of a power value in the second set of power values to a maximal aerobic power (MAP) of the user and contributes to the second metric power component when the power value in the second set of power values is greater than the FTP of the user and is within a tolerance of the MAP of the user,
wherein the second metric cadence component is a sum of second metric cadence component data points, where a second metric cadence component data point is derived as a function of a ratio of a power value in the second set of power values to the MAP of the user and contributes to the second metric cadence component when a cadence value in the first set of cadence values is greater than the optimal cadence of the user, and
wherein the MAP of the user is the maximum power that can be maintained by the user for a duration of about 5 minutes.

11. The method of claim 10, wherein calculating the fourth metric comprises:
receiving, by the processor of the computer, measurements of torque generated by the user the user during the activity;
calculating a set of torque values by averaging the torque generated by the user during the activity in a time window having the fourth duration at each time point in the set of periodic time points during the activity; and
calculating a sum of a fourth metric power component, a fourth metric cadence component, and a fourth metric torque component,
wherein the fourth power component comprises a sum of fourth metric power component data points, where a fourth metric power component data point is derived as a function of a ratio of a power value in the fourth set of power values to a neuromuscular (NM) power of the user and contributes to the fourth metric power component when the power value in the fourth set of power values is greater than a predetermined fraction of the NM power of the user,
wherein the fourth metric cadence component is a sum of fourth metric cadence component data points, where a fourth metric cadence component data point is derived as a function of a ratio of a power value in the fourth set of power values to the NM power of the user and contributes to the fourth metric cadence component when a cadence value in the second set of cadence values is greater than the predefined optimal cadence of the user,
wherein the fourth metric torque component is a sum of fourth metric torque component data points, where each fourth metric torque component data point is derived as a function of a ratio of a power value in the fourth set of power values to the NM power of the user and contributes to the fourth metric torque component when (i) a torque value in the set of torque values at a given time point in the set of periodic time points is greater than a threshold torque of the user and (ii) a cadence value in the second set of cadence values at the given time point is within a tolerance of the predefined optimal cadence of the user or is less than the predefined optimal cadence of the user, and
wherein the NM power of the user is the maximum power that can be maintained by the user for a duration of about 5 seconds;
optionally further comprising:
calculating, by the processor of the computer, the torque threshold of the user as a function of the predefined optimal cadence of the user and the FTP of the user.

12. The method of claim 11, wherein
calculating the first metric further comprises multiplying the sum of first metric data points by a first multiplier;
calculating the second metric further comprises multiplying the sum of second metric data points by a second multiplier;
calculating the third metric further comprises multiplying the sum of third metric data points by a third multiplier; and
calculating the fourth metric further comprises multiplying the sum of fourth metric data points by a fourth multiplier,
wherein the second multiplier is greater than the first multiplier, the third multiplier is greater than the second multiplier, and the fourth multiplier is greater than the third multiplier.

13. The method of claim 1, further comprising:
calculating, by the processor of the computer, an intensity density (ID) score for the activity by summation of the first metric, the second metric, the third metric, and the fourth metric, the ID score being indicative of overall accumulated physiological strain experienced by the user during the activity; and
displaying, by the display of the computer, the ID score to the user,
optionally further comprising:
displaying, by the display of the computer, the portion of the ID score respectively attributed to by the first metric, the second metric, the third metric, and the fourth metric.

14. The method of claim 1, wherein the first metric, the second metric, the third metric, and the fourth metric are not derived as a function of a heart rate of the user during the activity.

15. A system for quantitation of physiological strain experienced by a user during a cycling activity, the system comprising:
a bicycle;
a power meter configured to measure power generated by a user operating the bicycle and to output a signal indicative of the power measured thereby;
a cadence sensor configured to measure a cadence of the user operating the bicycle and to output a signal indicative of the cadence measured thereby; and
a computer including a display, a processor, and memory storing instructions for execution by the processor, the processor being configured by the instructions to:
receive measurements of power exerted by the user during the activity;
calculate a first metric indicative of sub-threshold and threshold level aerobic strain experienced by the user during the activity;
calculate a second metric indicative of suprathreshold level strain experienced by the user during the activity;
calculate a third metric indicative of anaerobic strain experienced by the user during the activity;
calculate a fourth metric indicative of neuromuscular strain experienced by the user during the activity, where the first, second, third, and fourth metrics are each derived as a function of the measurements of the power exerted by the user during the activity; and
command the display to display indicia of the first metric, the second metric, the third metric, and the fourth metric.
